# EUROPÄISCHE PATENTSCHRIFT

(11) **EP 2 929 863 B1**
(45) Veröffentlichungstag und Bekanntmachung des Hinweises auf die Patenterteilung: **21.02.2018**
(21) Anmeldenummer: 15000986.8
(22) Anmeldetag: 07.04.2015
(51) Int. Cl.: A61F 13/14, A61F 5/03, A61F 5/30, A61F 5/32

(54) **DRUCKVERBAND**
COMPRESSION BANDAGE
BANDE COMPRESSIVE

(30) Priorität: 07.04.2014 DE 102014004988
(43) Veröffentlichungstag der Anmeldung: 14.10.2015
(73) Patentinhaber: Östreicher, Ulrich, 37276 Meinhard (DE)
(72) Erfinder: Östreicher, Ulrich, 37276 Meinhard (DE)
(74) Vertreter: Gittinger, Andreas

(56) Entgegenhaltungen:
- EP-A2- 1 859 762
- WO-A1-2006/069548
- WO-A1-2008/142186
- US-A- 3 561 442
- US-A1- 2001 037 076
- US-B1- 6 516 804

## Beschreibung

Die Erfindung betrifft einen Druckverband zum Andrücken eines Kompressoriums oder einer Kompresse an eine Brusthälfte eines Patienten nach einer Herzschrittmacherimplantation gemäß dem Oberbegriff des Anspruchs 1.
Nach einer Operation, bei der einem Patienten ein Herzschrittmacher oder ein anderes das Herz unterstützendes Gerät eingesetzt wird, wird die Operationswunde oftmals vernäht und mit einem Klebeverband versorgt. Häufig wird über den Klebeverband noch ein Sandsack gelegt, um einen Druck auf die Operationswunde zu erzeugen. Damit soll eine Ödembildung verhindert oder wenigstens reduziert werden. Der Patient ist somit gezwungen, die ganze Zeit zu liegen, damit der Sandsack auf die Operationswunde drücken und nicht verrutschen kann. Dies ist natürlich für den Patienten ausgesprochen anstrengend und unkomfortabel.
Die WO 2006/069 548 A1 beschreibt einen Verband zur Blutstillung nach einer Herzschrittmacheroperation. Er ist mit einem manuell aufpumpbaren Luftbeutel ausgerüstet. Ferner weist er ein Befestigungsmittel zur Befestigung des Luftbeutels am Körper des Patienten auf.
In der US 3561 442 ist eine Bandage zum Gebrauch nach einer Brustamputation offenbart. Sie wird nach der Operation über einem Verband, welcher die Operationswunde abdeckt, aufgebracht. Die Bandage soll den Verband gegen vor einem Verrutschen sichern.

Eine Orthese, mit der ebenfalls nach einer Brustamputation eine Flüssigkeitsansammlung aus einem Gewebe des Patienten eliminiert werden soll, wird in der WO 2008/142 186 A1 erwähnt.
Einen Herz-/Brustgurt, der einen Druck im Bereich eines Brustkorbs des Patienten erzeugen soll, beschreibt die US 6516 804 B1. Der Druck auf den Brustkorb kann so stark sein, dass im Einzelfall auf Schulterbänder verzichtet werden kann.
In der US 2001/003 70 76 A1 wird ein korsettartiges Gewand, das für eine Behandlung unter Druck und Temperatureinwirkung getragen wird, beschrieben. Es soll dem Patienten bei Schmerzen und Unbehagen Erleichterung verschaffen.
Einen Verband zur Kompression des Thorax beschreibt die EP 1 859 762 A2. Er wird vorwiegend dann eingesetzt, wenn der Brustbereich des Patienten vor unerwünschten Bewegungen geschützt werden soll, wie beispielsweise bei Rippenbrüchen.
Die Erfindung hat die Aufgabe, einen Druckverband zu schaffen, mit dem auf der Brust ein erforderlicher Anpressdruck erzeugt und einem Patienten mehr Bewegungsfreiheit eingeräumt wird, insbesondere auch beim Atmen.
Die Erfindung löst die gestellte Aufgabe mit einem Druckverband gemäß Anspruch 1. Der Druckverband erzeugt somit den erforderlichen Anpressdruck, mit dem das Kompressorium flächig gegen eine Operationswunde gedrückt wird. Durch die flächige Anpressung kann das Kompressorium sowohl gegen eine wundnaht der Operationswunde als auch gegen die Umgebung der Wundnaht gedrückt werden. Auf diese Weise wird die Gefahr der Bildung eines Ödems oder einer Entzündung reduziert. Die Anpressung des Schulterbands ist vorzugsweise höher als die Anpressung des Brustbandes. Dadurch wird die Atmung des Patienten nicht unnötig behindert, wobei gleichzeitig der Brustverband seine Position am Patienten zuverlässig beibehält. Da auf konventionelle Pflaster verzichtet werden kann, ist der Druckverband für den Patienten auch hautschonend. Zudem ist eine Nachjustierung des Kompressoriums oder der Kompresse jederzeit möglich. Eine nochmalige unangenehme Neuanlage eines Pflasterverbands entfällt also.

Mit den an den Enden des Brustbandes befindlichen Klettpolstern kann das Brustband an der Vorderseite des Patienten verschlossen werden. Da die Klettpolster auf den gegenüberliegenden Seiten des Brustbandes angeordnet sind, lässt es sich unabhängig davon, ob das Schulterband über die linke oder die rechte Brusthälfte geführt wird, gut verschließen. Die doppelseitig wirkenden Klettpolster können zur optimalen Anlage des Druckverbandes am Patienten individuell am Brustband und/oder am Schulterband positioniert werden. Wenn der Herzschrittmacher an der rechten Brusthälfte implantiert wurde, so kann der Druckverband durch einfaches Wenden alternativ über die rechte Brusthälfte geführt werden. Der Druckverband kann aus einem Vlies, einem Vliesverbund oder aus einem textilen Gewebe hergestellt sein.

Um das Anlegen des Druckverbands am Patienten in kurzer Zeit zu ermöglichen, kann das Schulterband mit seinem einen Ende am Brustband angenäht sein. Die Nahtstelle kann sich vorteilhafterweise am Rücken des Patienten befinden.

Damit das Kompressorium optimal gegen die Operationswunde gedrückt wird, kann das Schulterband das Kompressorium vollständig überdecken. Das Schulterband kann mit seinen beiden Längsrändern das Kompressorium überlappen, so dass das Kompressorium vollständig vom Schulterband verdeckt ist und nicht unbeabsichtigt von der Operationswunde herunterrutschen kann.

Das Brustband kann das Kompressorium teilweise überdecken. Zweckmäßigerweise überdeckt das Brustband den zu den Beinen des Patienten hin gewandten Bereich des Kompressoriums. Somit verhindert das Kompressorium, dass das Brustband zu den Schultern des Patienten hin verrutschen kann. Außerdem kann das Brustband auf diese Weise das Kompressorium ebenfalls gegen die Operationswunde drücken.

An dem Schulterband kann ein Befestigungselement zur Befestigung des Kompressoriums oder der Kompresse an dem Schulterband vorgesehen sein. Die Kompresse kann eine Kühlkompresse sein. Es ist jedoch auch möglich, dass man mehrere Mullkompressen oder andere damit vergleichbare Anpressdruckverstärker mit dem Befestigungselement am Schulterband fixiert.

Das Befestigungselement kann ein Klettpolster oder eine Klebefläche sein. Somit kann die Kompresse schnell am Schulterband angebracht oder von ihm entfernt werden.

Das Befestigungselement kann ferner ein Band sein, das mit seinem einen Ende am Schulterband befestigt ist und an seinem anderen Ende ein Klettpolster aufweist, an dem das Kompressorium oder die Kompresse befestigt werden kann.

Alternativ kann an dem Schulterband eine Tasche zur Aufnahme des Kompressoriums angebracht sein. Somit lässt sich das Kompressorium am Schulterband anordnen, bevor der Druckverband an den Patienten angelegt wird. Dies hilft ebenfalls den Druckverband in kürzester Zeit anzulegen.

Damit das Kompressorium individuell am Patienten positioniert werden kann, kann die Tasche entlang des Schulterbands verschoben werden. Zu diesem Zweck kann eine Schlaufe an die Tasche genäht und das Schulterband durch die Schlaufe geführt sein.

Mit dem Befestigungselement und der Tasche kann der Druckverband mit der Kompresse vorkonfektioniert werden.

Das Schulterband kann schräg am Brustband angenäht sein, so dass das Schulterband und das Brustband einen Winkel von weniger als 90° einschließen. Dadurch kann das Schulterband positionsstabiler über die Schulter geführt werden, so dass es nicht so leicht von der Schulter rutschen kann. In der Praxis hat sich ein Winkelbereich zwischen 50° und 85° besonders gut bewährt.

Alternativ ist es jedoch auch möglich, das Schulterband in einem Winkel von 90° am Brustband anzunähen.

Um den Tragekomfort für den Patienten zu erhöhen, kann das Schulterband im Halsbereich des Patienten eine Ausnehmung aufweisen. Dies ist besonders dann nützlich, wenn mit dem Schulterband ein besonders hoher Anpressdruck erzeugt werden muss, da dann das Schulterband möglichst nah am Hals vorbeigeführt werden muss. Durch den Ausschnitt kann das Schulterband den Patienten am Hals kaum stören.

Das Brustband kann an der am Patienten anliegenden Seite mit mindestens einem hautverträglichen Klebepolster ausgestattet sein. Dadurch wird das Brustband am Körper zuverlässig fixiert, so dass es nicht in Richtung der Schulter rutschen kann und eine zuverlässige Anpressung des Kompressoriums gewährleistet ist. Das Klebepolster kann auf der Basis eines Poliacrylat-Klebers aufgebaut sein. Das Klebepolster kann vorzugsweise mikropunktuell aufgebracht werden um ein vollflächiges Verkleben auf der Haut des Patienten zu vermeiden. Aus demselben Grund kann das Klebepolster alternativ auch kleine Streifen aufweisen.

Ebenfalls, um ein vollflächiges Verkleben auf der Haut des Patienten zu vermeiden, kann das Brustband alternativ auf der am Patienten anliegenden Seite mit einer Haftschicht ausgerüstet sein. Die Haftschicht kann beispielsweise aus Silikon oder einer Polyurethan-Beschichtung aufgebaut sein. Auch in diesem Fall ist es sinnvoll, wenn die Haftschicht mikropunktuell oder in Form von kleinen Streifen aufgebaut ist.

Um Infektionen in der Operationswunde zu vermeiden, kann das Brustband und/oder das Schulterband eine Barrierelage, die den Durchgang von Bakterien blockiert, aufweisen.

Das Brustband und/oder das Schulterband kann auch mit einer semipermeablen Folie ausgerüstet sein. Auf diese Weise ist das Brustband und/oder das Schulterband für Luft und Hautfeuchtigkeit durchlässig. Außerdem schützt die semipermeable Folie die Operationswunde vor dem Eintritt von Bakterien oder von Nässe, die von außen kommt. Dadurch wird der Heilungsprozess unterstützt.

Die an dem einen der beiden Enden auf den gegenüberliegenden Seiten des Brustbandes angebrachten Klettpolster können vorzugsweise angenäht sein.

Nachfolgend werden verschiedene Ausführungsbeispiele des erfindungsgemäßen Druckverbands anhand der beiliegenden Zeichnungen näher erläutert.

Im Einzelnen zeigen:
- Fig. 1: eine Vorderansicht eines an einem Patienten angelegten Druckverbandes;
- Fig. 2: eine Rückansicht des Druckverbandes aus Fig. 1;
- Fig. 3: eine Vorderansicht einer zweiten Ausführungsform des an den Patienten angelegten Druckverbandes;
- Fig. 4: eine Rückansicht des Druckverbandes aus Fig. 3;
- Fig. 5: eine Schnittansicht durch ein Kompressorium.

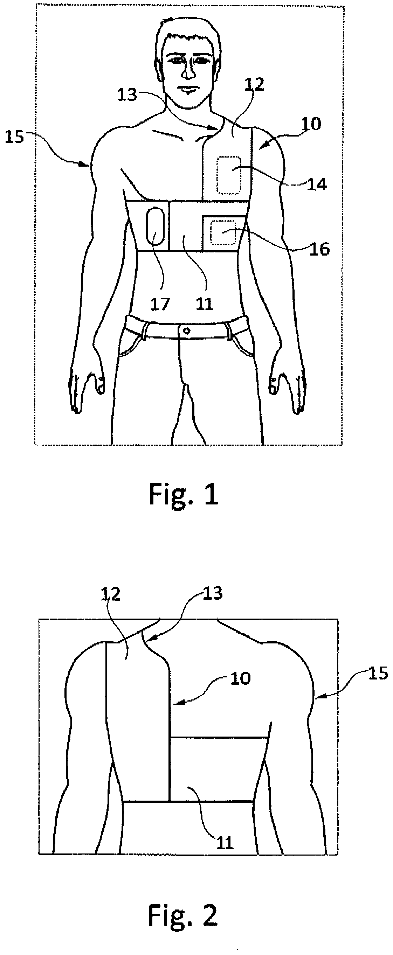

Die Fign. 1 und 2 zeigen einen Druckverband 10 mit einem Brustband 11. An dem Brustband 11 ist ein Schulterband 12 angenäht. Das Schulterband 12 überdeckt ein darunter angeordnetes Kompressorium 14, das auf einer Brusthälfte eines Patienten 15 angeordnet ist, vollständig, so dass die Ränder des Schulterbands 12 zum Kompressorium 14 beabstandet sind. Dadurch wird das Kompressorium 14 optimal gegen eine Operationswunde gedrückt und kann nicht unerwünschterweise von der Operationswunde herunterrutschen. Weil das Kompressorium sowohl gegen eine Wundnaht der Operationswunde als auch gegen die Umgebung der Operationswunde drückt, wird die Gefahr der Bildung eines Ödems oder einer Entzündung reduziert.
Das Schulterband 12 ist im Halsbereich des Patienten 15 mit einer Ausnehmung 13 versehen. Durch die Ausnehmung 13 kann das Schulterband 12 den Patienten 15 nicht im Bereich seines Halses stören.
Das Brustband 11 ist um einen Brustkorb des Patienten 15 herumgeführt. Es dient in erster Linie der Fixierung des gesamten Druckverbandes am Patienten 15 und der Aufrechterhaltung der Spannung des Schulterbands 12. Dadurch kann das Schulterband 12 das Kompressorium 14 mit einem bestimmten Druck gegen die Operationswunde drücken. Das Brustband 11 weist ein Klettpolster 17 auf. Auf der gegenüberliegenden verdeckten Seite des Brustbandes 11 befindet sich ebenfalls ein hier nicht näher erkennbares Klettpolster. Mit dem Klettpolster 17 und dem hier nicht erkennbaren Klettpolster kann das Brustband verschlossen werden je nachdem, ob das Schulterband 12 über die linke oder die rechte Brusthälfte des Patienten 15 geführt wird. Wenn das Schulterband 12 über die andere Schulter geführt werden soll, muss der Druckverband 10 lediglich gewendet werden.
Das Schulterband 12 wird an der Vorderseite des Patienten 15 von seiner Schulter kommend hinter dem Brustband 11 durchgeführt und dann am unteren Rand des Brustbandes 11 nach oben umgeschlagen und schließlich mit einem doppelseitig wirkenden Klettpolster 16 an der nach vorne weisenden Seite des Brustbands 11 befestigt. Das doppelseitig wirkende Klettpolster 16 kann abhängig von den Körpermaßen des Patienten 15 am Schulterband 12 und am Brustband 11 angeordnet werden.

Das Schulterband 12 ist am Brustband 11 mit einem Winkel von 90° angenäht.

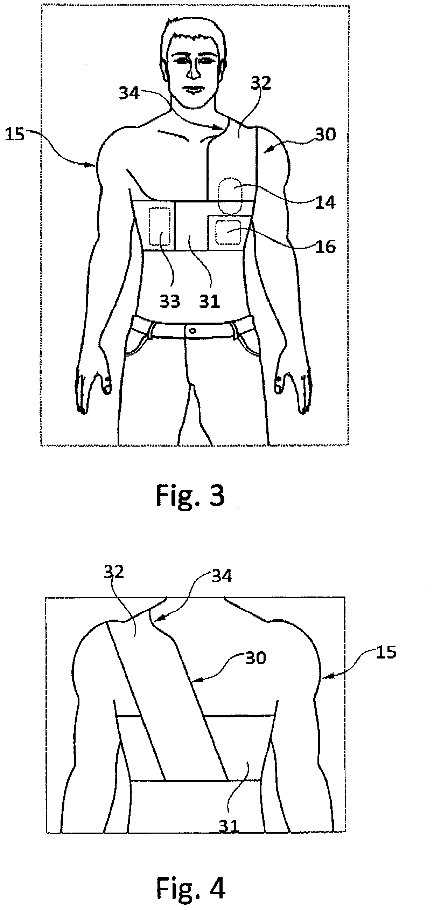

Die Fign. 3 und 4 zeigen einen Druckverband 30 mit einem Brustband 31 und einem Schulterband 32. Dass Schulterband 32 ist unter einem Winkel von weniger als 90° am Brustband 31 angenäht.

Das Brustband 31 überdeckt das Kompressorium 14 teilweise. Auf diese Weise verhindert das Kompressorium 14 auch, dass das Brustband zu den Schultern des Patienten 15 hin verrutschen kann. Außerdem leistet das Brustband 31 auf diese Weise ebenfalls einen Beitrag zum Anpressdruck, mit dem das Kompressorium 14 gegen die Operationswunde gedrückt wird.

Das Brustband 31 ist mit einem ebenfalls doppelseitig wirkenden Klettpolster 33 verschlossen. Das doppelseitig wirkende Klettpolster 33 ermöglicht es das Brustband 31 zu verschließen unabhängig davon, ob das Schulterband 32 über die linke oder die Rechte Brusthälfte des Patienten 15 geführt wird.

Das Schulterband 32 ist zur Steigerung des Tragekomforts mit einer Ausnehmung 34 versehen.

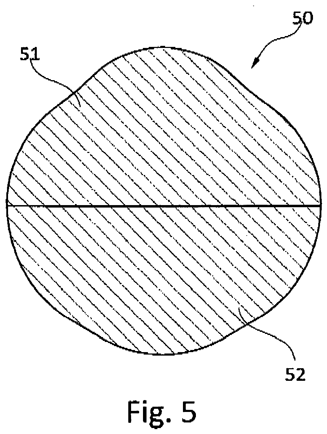

Fig. 5 zeigt das Kompressorium 50, welches zwei unterschiedlich gewölbte Hälften 51 und 52 aufweist. Durch die unterschiedlich gewölbten Hälften 51 und 52 besteht eine weitere Möglichkeit, den Anpressdruck des Kompressoriums 50 auf die Operationswunde individuell an die Erfordernisse des Patienten anzupassen.

### BEZUGSZEICHENLISTE

- 10: Druckverband
- 11: Brustband
- 12: Schulterband
- 13: Ausnehmung
- 14: Kompressorium
- 15: Patient
- 16: Klettpolster
- 17: Klettpolster

- 30: Druckverband
- 31: Brustband
- 32: Schulterband
- 33: Klettpolster
- 34: Ausnehmung

- 50: Kompressorium
- 51: Hälfte
- 52: Hälfte

## Patentansprüche

1. Druckverband (10, 30) zum Andrücken eines Kompressoriums (14, 50) oder einer Kompresse an eine Brusthälfte eines Patienten (15) nach einer Herzschrittmacherimplantation mit einem um einen Brustkorb geführten Brustband (11, 31), wobei an einem der beiden Enden des Brustbandes (11, 31) auf seinen gegenüberliegenden Seiten einenfalls Klettpolster (17) angebracht sind, und einem um eine Schulter geführten Schulterband (12, 32), das mit seinem einen Ende zwischen den beiden Enden des Brustbandes (11, 31) am Brustband (11, 31) außermittig befestigt ist, **dadurch gekennzeichnet, dass** einenfalls zur Fixierung des anderen Endes des Schulterbandes (12, 32) am Brustband (11, 31) ein doppelseitig wirkendes Klettpolster (16) vorgesehen ist und anderenfalls anstelle der Klettpolster (17) nur auf einer der gegenüberliegenden Seiten des Brustbandes (11, 31) an einem seiner beiden Enden ein doppelseitig wirkendes Klettpolster (33) angebracht ist und zur Fixierung des anderen Endes des Schulterbandes (12, 32) am Brustband (11, 31) auch das doppelseitig wirkende Klettpolster (16) vorgesehen ist so dass in beiden Fällen das Schulterband (12, 32) durch Wenden des Druckverbands (10, 30) entweder über die linke oder die rechte Brusthälfte führbar ist.

2. Druckverband (10, 30) nach Anspruch 1, **dadurch gekennzeichnet, dass** das Schulterband (12, 32) mit seinem einen Ende am Brustband (11, 31) angenäht ist.

3. Druckverband (10, 30) nach Anspruch 1 oder 2, **dadurch gekennzeichnet, dass** das Schulterband (12, 32) das Kompressorium (14, 50) vollständig überdeckt.

4. Druckverband (10, 30) nach einem der Ansprüche 1 bis 3, **dadurch gekennzeichnet, dass** das Brustband (11, 31) das Kompressorium (14, 50) wenigstens teilweise überdeckt.

5. Druckverband (10, 30) nach einem der Ansprüche 1 bis 3, **dadurch gekennzeichnet, dass** an dem Schulterband (12, 32) ein Befestigungselement zur Befestigung des Kompressoriums (14, 50) oder der Kompresse am Schulterband (12, 32) vorgesehen ist.

6. Druckverband (10, 30) nach Anspruch 5, **dadurch gekennzeichnet, dass** das Befestigungselement ein Klettpolster oder eine Klebefläche ist.

7. Druckverband (10, 30) nach Anspruch 5 oder 6, **dadurch gekennzeichnet, dass** das Befestigungselement ein Band ist, das mit seinem einen Ende am Schulterband (12, 32) befestigt ist und an seinem anderen Ende ein Klettpolster aufweist.

8. Druckverband (10, 30) nach einem der Ansprüche 1 bis 7, **dadurch gekennzeichnet, dass** an dem Schulterband (12, 32) eine Tasche zur Aufnahme des Kompressoriums (14, 50) angebracht ist.

9. Druckverband (10, 30) nach Anspruch 8, **dadurch gekennzeichnet, dass** die Tasche entlang des Schulterbands (12, 32) verschiebbar angeordnet ist.

10. Druckverband (10, 30) nach einem der Ansprüche 2 bis 9, **dadurch gekennzeichnet, dass** das Schulterband (12, 32) schräg am Brustband (11, 31) angenäht ist, so dass das Schulterband (12, 32) und das Brustband (11, 31) einen Winkel von weniger als 90° einschließen.

11. Druckverband (10, 30) nach einem der Ansprüche 1 bis 10, **dadurch gekennzeichnet, dass** das Schulterband (12, 32) im Halsbereich des Patienten (15) eine Ausnehmung (13) aufweist.

12. Druckverband (10, 30) nach einem der Ansprüche 1 bis 11, **dadurch gekennzeichnet, dass** das Brustband (11, 31) auf der am Patienten (15) anliegenden Seite mit einer Haftschicht versehen ist.

13. Druckverband (10, 30) nach einem der Ansprüche 1 bis 12, **dadurch gekennzeichnet, dass** das Brustband (11, 31) auf der am Patienten (15) anliegenden Seite mit mindestens einem hautverträglichen Klebepolster ausgestattet ist.

14. Druckverband (10, 30) nach einem der Ansprüche 1 bis 13, **dadurch gekennzeichnet, dass** das Brustband (11, 31) und/oder das Schulterband (12, 32) eine Barrierelage, die den Durchgang von Bakterien blockiert, aufweist.

15. Druckverband (10, 30) nach einem der Ansprüche 1 bis 14, **dadurch gekennzeichnet, dass** die an dem einen der beiden Enden des Brustbrandes (11, 31) angeordneten Klettpolster (17) angenäht sind.

## Claims

1. Compression bandage (10, 30) for pressing a compressorium (14, 50) or a compress against a side of the chest of a patient (15) after a pacemaker implant operation, with a chest strap (11, 31) passing around a ribcage, wherein in one case Velcro pads (17) are attached to opposite sides of one of the two ends of the chest strap (11, 31), and a shoulder strap (12, 32) passing around a shoulder, one end of which shoulder strap is fastened to the chest strap (11, 31) between the two ends of the chest strap (11, 31) and off-center thereon, **characterized in that** in one case a Velcro pad (16) with double-sided effect is provided for fastening the other end of the shoulder strap (12, 32) to the chest strap (11, 31), and otherwise, instead of the Velcro pads (17) on only one of the opposite sides of the chest strap (11, 31), a Velcro pad (33) with double-sided effect is attached to one of the two ends thereof, and the Velcro pad (16) with double-sided effect is also provided to fasten the other end of the shoulder strap (12, 32) to the chest strap (11, 31), so that in both cases the shoulder strap (12, 32) can be passed either over the left or the right side of the chest by turning the compression bandage (10, 30).

2. Compression bandage (10, 30) according to Claim 1, **characterized in that** one end of the shoulder strap (12, 32) is sewn onto the chest strap (11, 31).

3. Compression bandage (10, 30) according to Claim 1 or 2, **characterized in that** the shoulder strap (12, 32) completely covers the compressorium (14, 50).

4. Compression bandage (10, 30) according to any one of Claims 1 to 3, **characterized in that** the chest strap (11, 31) at least partially covers the compressorium (14, 50).

5. Compression bandage (10, 30) according to any one of Claims 1 to 3, **characterized in that** a fastening element is provided on the shoulder strap (12, 32) for fastening the compressorium (14, 50) or the compress to the shoulder strap (12, 32).

6. Compression bandage (10, 30) according to Claim 5, **characterized in that** the fastening element is a Velcro pad or an adhesive surface.

7. Compression bandage (10, 30) according to Claim 5 or 6, **characterized in that** the fastening element is a strap, one end of which is fastened to the shoulder strap (12, 32), and the other end of which is furnished with a Velcro pad.

8. Compression bandage (10, 30) according to any one of Claims 1 to 7, **characterized in that** a pouch for accommodating the compressorium (14, 50) is attached to the shoulder strap (12, 32).

9. Compression bandage (10, 30) according to Claim 8, **characterized in that** the pouch is arranged so as to be displaceable along the shoulder strap (12, 32).

10. Compression bandage (10, 30) according to any one of Claims 2 to 9, **characterized in that** the shoulder strap (12, 32) is sewn onto the chest strap (11, 31) at an angle, so that the shoulder strap (12, 32) and the chest strap (11, 31) form an angle less than 90°.

11. Compression bandage (10, 30) according to any one of Claims 1 to 10, **characterized in that** the shoulder strap (12, 32) has a recess (13) in the neck area of the patient (15).

12. Compression bandage (10, 30) according to any one of Claims 1 to 11, **characterized in that** the chest strap (11, 31) is provided with an adhesive layer on the side that is in contact with the patient (15).

13. Compression bandage (10, 30) according to any one of Claims 1 to 12, **characterized in that** the chest strap (11, 31) is provided with at least one skin-tolerable adhesive pad on the side that is in contact with the patient (15).

14. Compression bandage (10, 30) according to any one of Claims 1 to 13, **characterized in that** the chest strap (11, 31) and/or the shoulder strap (12, 32) has/have a barrier layer that blocks the passage of bacteria.

15. Compression bandage (10, 30) according to any one of Claims 1 to 14, **characterized in that** the Velcro pads (17) arranged on one of the two ends of the chest strap (11, 31) are sewn thereto.

## Revendications

1. Pansement compressif (10,30) pour application d'un élément de compression (14,50) ou d'une compresse sur une moitié de poitrine d'un patient (15) après une implantation de stimulateur cardiaque avec une bande pectorale (11,31) guidée autour de la cage thoracique, des tampons Velcro (17) étant disposés d'une part sur une des deux extrémités de la bande pectorale (11,31) sur ses côtés opposés et une bandoulière (12,32) guidée autour d'une épaule qui est fixée excentrée sur la bande pectorale (11,31) avec une de ses extrémités entre les deux extrémités de la bande pectorale (11,31), **caractérisé en ce que** d'une part pour la fixation de l'autre extrémité de la bandoulière (12,32) un tampon Velcro (16) agissant des deux côtés est prévu sur la bande pectorale (11,31) et d'autre part un tampon Velcro (33) agissant des deux côtés est disposé à la place du tampon Velcro (17) uniquement sur un des côtés opposés de la bande pectorale (11,31) sur une de ses deux extrémités et le tampon Velcro (16) agissant des deux côtés est également prévu pour la fixation de l'autre extrémité de la bandoulière (12,32) sur la bande pectorale (11,31) de telle sorte que dans les deux cas, la bandoulière (12,32) peut être déplacée en tournant le pansement compressif (10,30), soit sur la moitié gauche soit sur la moitié droite de la poitrine.

2. Pansement compressif (10,30) selon la revendication 1, **caractérisé en ce que** la bandoulière (12,32) est cousue à la bande pectorale (11,31) avec une de ses extrémités.

3. Pansement compressif (10,30) selon la revendication 1 ou 2, **caractérisé en ce que** la bandoulière (12,32) recouvre complètement l'élément de compression (14,50).

4. Pansement compressif (10,30) selon l'une quelconque des revendications 1 à 3, **caractérisé en ce que** la bande pectorale (11,31) recouvre au moins en partie l'élément de compression (14,50).

5. Pansement compressif (10,30) selon l'une quelconque des revendications 1 à 3, **caractérisé en ce que** sur la bandoulière (12,32), un élément de fixation est prévu pour la fixation de l'élément de compression (14,50) ou de la compresse à la bandoulière (12,32).

6. Pansement compressif (10,30) selon la revendication 5, **caractérisé en ce que** l'élément de fixation est un tampon Velcro ou une surface adhésive.

7. Pansement compressif (10,30) selon la revendication 5 ou 6, **caractérisé en ce que** l'élément de fixation est une bande, qui est fixée avec une de ses extrémités à la bandoulière (12,32) et comporte un tampon Velcro à son autre extrémité.

8. Pansement compressif (10,30) selon l'une quelconque des revendications 1 à 7, **caractérisé en ce qu'**une poche pour recevoir l'élément de compression (14,50) est disposée sur la bandoulière (12,32).

9. Pansement compressif (10,30) selon la revendication 8, **caractérisé en ce que** la poche est disposée pouvant être déplacée le long de la bandoulière (12,32).

10. Pansement compressif (10,30) selon l'une quelconque des revendications 2 à 9, **caractérisé en ce que** la bandoulière (12,32) est cousue en diagonale sur la bande pectorale (11,31) de telle sorte que la bandoulière (12,32) et la bande pectorale (11,31) forment un angle inférieur à 90°.

11. Pansement compressif (10,30) selon l'une quelconque des revendications 1 à 10, **caractérisé en ce que** la bandoulière (12,32) comporte un évidement (13) dans la zone du cou du patient (15).

12. Pansement compressif (10,30) selon l'une quelconque des revendications 1 à 11, **caractérisé en ce que** la bande pectorale (11,31) est dotée d'une couche adhésive sur le côté reposant sur le patient (15).

13. Pansement compressif (10,30) selon l'une quelconque des revendications 1 à 12, **caractérisé en ce que** la bande pectorale (11,31) est équipée sur le côté reposant sur le patient (15) d'au moins un tampon Velcro toléré par la peau.

14. Pansement compressif (10,30) selon l'une quelconque des revendications 1 à 13, **caractérisé en ce que** la bande pectorale (11,31) et/ou la bandoulière (12,32) comportent une couche barrière qui bloque le passage de bactéries.

15. Pansement compressif (10,30) selon l'une quelconque des revendications 1 à 14, **caractérisé en ce que** les tampons Velcro (17) disposés sur l'une des deux extrémités de la bande pectorale (11,31), sont cousus.
